# NEW EUROPEAN PATENT SPECIFICATION

(11) **EP 1 180 978 B2**
(45) Date of publication and mention of the opposition decision: **17.11.2010**
(45) Mention of the grant of the patent: 11.05.2005
(21) Application number: 00932801.4
(22) Date of filing: 30.05.2000
(51) Int. Cl.: A61B 17/56, A61F 2/44

(54) **APPARATUS FOR TREATING DISC HERNIATION**
VORRICHTUNG ZUR BEHANDLUNG VON HERNIA AN BANDSCHEIBEN
APPAREIL PERMETTANT DE TRAITER LES HERNIES DISCALES

(30) Priority: 28.05.1999 US 322516
(43) Date of publication of application: 27.02.2002
(73) Proprietor: Ferree, Bret A., Cincinnati, OH 45208 (US)
(72) Inventor: Ferree, Bret A., Cincinnati, OH 45208 (US)
(74) Representative: Wilson, Gary
(86) International application number: PCT/US2000/014708
(87) International publication number: WO 2001/010316

(56) References cited:
- EP-A1- 0 700 671
- WO-A-95/31946
- WO-A-97/26847
- WO-A-98/20939
- WO-A-99/02108
- WO-A1-95/34331
- WO-A1-99/61084
- FR-A- 2 639 823
- US-A- 4 904 260
- US-A- 5 192 326
- US-A- 5 534 028
- US-A- 5 645 597
- US-A- 5 702 454
- US-A- 5 746 765
- US-A- 5 824 093
- US-A- 5 846 261

## Description

### Field of the Invention

This invention relates generally to devices for occluding intervertebral disc defects.

### Background of the Invention

Several hundred thousand patients undergo disc operations each year. Approximately five percent of these patients will suffer recurrent disc herniation, which results from a void or defect which remains in the outer layer (annulus fibrosis) of the disc after surgery involving partial discectomy.

WO-A-9820939 relates to a method and related composition and apparatus for repairing a tissue site. The method involves the use of a curable polyurethane biomaterial composition having a plurality of parts adapted to be mixed at a time of use in order to provide flowable composition and to initiate cure. The flowable composition can be delivered using minimally invasive means to a tissue site and they are fully cured to provide a permanent and biocompatible prosthesis for repair of the tissue site. Furthermore, there is provided a mould apparatus for example in the form of a balloon or tubular cavity, for receiving a biomaterial composition and a method for delivering and filling the mould apparatus with a curable composition *in situ* to provide a prosthesis for tissue repair.

US-A-5645597 relates to a method for replacing a nucleus pulposus of an intervertebral disc. The method is achieved by removing the nucleus pulposus from the intervertebral disc to create a space defined by an inner wall of an annulus fibrosis. A flexible prosthetic disc is then inserted within the space formerly occupied by the nucleus pulposus and the prosthetic disc is subsequently filled with a gel.

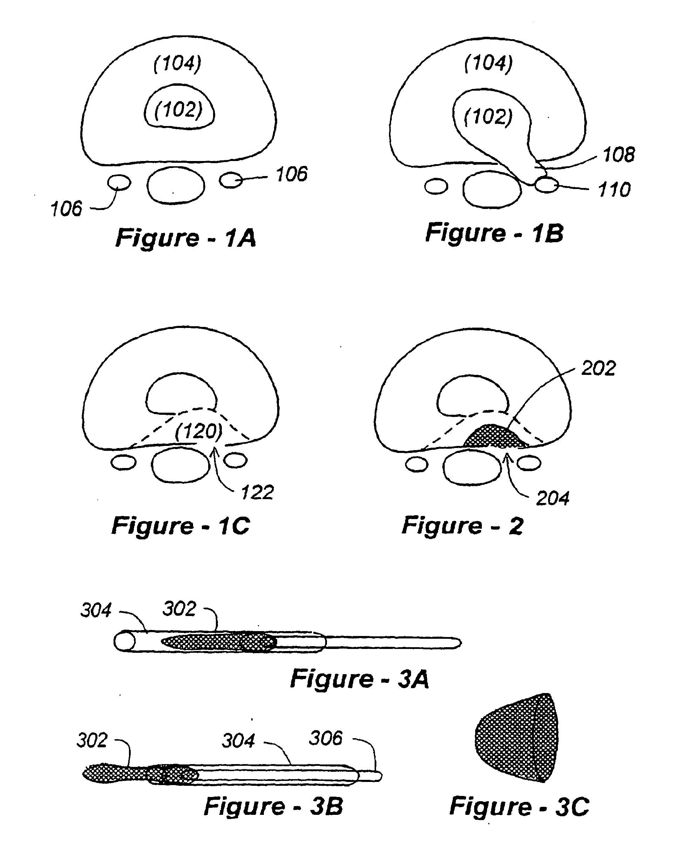

Reference is made to Figure 1A, which illustrates a normal disc as viewed from the feet of a patient up toward the head. The nucleus pulposus 102 is entirely surrounded by the annulus fibrosis 104 in the case of healthy anatomy. Also shown in this cross section is the relative location of the nerves 106. Figure 1B illustrates the case of the herniated disc, wherein a portion of the nucleus pulposus has ruptured through a defect in the annulus fibrosis, resulting in a pinched nerve 110. This results in pain and further complications, in many cases.

Figure 1C illustrates the post-operative anatomy following partial discectomy, wherein a space 120 remains adjacent a hole or defect in the annulus fibrosis following removal of the disc material. The hole 122 acts as a pathway for additional material to protrude into the nerve, resulting in the recurrence of the herniation. Since thousands of patients each year require surgery to treat this condition, with substantial implications in terms of the cost of medical treatment and human suffering, any solution to this problem would be welcomed by the medical community.

### Summary of the Invention

According to a first aspect of the present invention there is provided a device as defined in claim 1.

The subject invention resides in apparatus for treating disc herniation, which may be defined as the escape of nucleus pulposus through a void or defect in the annulus fibrosis of a spinal disc situated between upper and lower vertebra. In addition to preventing therelease of natural disc materials, the invention may also be used to retain bone graft for fusion, therapeutic and artificial disc replacement materials. The invention is particularly well suited to the minimization and prevention of recurrent disc herniation, in which case the defect is a hole or void which remains in the annulus fibrosis following disc operations involving partial discectomy.

In broad, general terms, to correct defects of this type, the invention provides a conformable device which assumes a first shape associated with insertion and a second shape or expanded shape to occlude the defect.

The device is preferably collapsible into some form for the purposes of insertion, thereby minimizing the size of the requisite incision while avoiding delicate surrounding nerves. Such a configuration also permits the use of instrumentation to install the device, including, for example, a hollow tube and a push rod to expel the device out of the sheath for use in occluding the disc defect.

A device according to the invention may further include one or more anchors to assist in permanently affixing the device with respect to the defect.

### Brief Description of the Drawings

FIGURE 1A is a cross section of a human disc exhibiting normal anatomy;
FIGURE 1B is a cross section used to illustrate a disc herniation;
FIGURE 1C is a drawing of a disc following a partial discectomy, showing how a space or void remains in the annulus fibrosis;
FIGURE 2 although not within the scope of the invention is a drawing which illustrates a flexible stent used to occlude a defect in the annulus fibrosis to minimize recurrent disc herniation;
FIGURE 3A although not within the scope of the invention is a drawing of an applicator used to insert the flexible mesh stent of Figure 2;
FIGURE 3B although not within the scope of the invention shows the applicator of Figure 3A with the stent partially expelled;
Figure 3C illustrates a fully expanded shape assumed by the device of Figure 2 following removal of the insertion tool;
FIGURE 4A although not within the scope of the invention illustrates the addition of optional peripheral anchors around the stent in the Figure 3C to assist in fixation;
FIGURE 4B although not within the scope of the invention is an end view of the device of Figure 4A including the peripheral anchors;
FIGURE 5 although not within the scope of the invention is a side-view drawing of the device of Figures 4A and 4B anchored into upper and lower vertebra bounding the herniated disc;
FIGURE 6A although not within the scope of the invention illustrates an optional distraction tool used to set the anchors of the device of Figures 4 and 5 into the vertebra;
FIGURE 6B shows how the distracting tool would be inserted into the device to effectuate distraction;
FIGURE 7A although not within the scope of the invention is a side-view drawing in partial cross-section illustrating the way in which notches may be made to adjoining vertebra to receive a device according to the invention;
FIGURE 7B although not within the scope of the invention is a drawing of a tool which may be used to form the notches depicted in Figure 7A;
FIGURE 7C although not within the scope of the invention illustrates the way in which a flexible body may be retained by the notches described with respect to Figures 7A and 7B;
FIGURE 8 although not within the scope of the invention illustrates an alternative orientation of a flexible body having a convex surface facing outwardly with respect to the wall of the disc being repaired;
FIGURE 9A although not within the scope of the invention illustrates how a device may be fixed with anchors that penetrate through the disc to be captured at the outer wall thereof;
FIGURE 9B although not within the scope of the invention illustrates an alternative use of anchors which remain within the body of the disc material and do not penetrate its outer wall;
FIGURE 9C although not within the scope of the invention illustrates an alternative method of fixation, wherein bone anchors are introduced into the vertebrae on either side of the disc in need of repair, as opposed to anchors deployed within or through the disc itself;
FIGURE 10A although not within the scope of the invention illustrates an alternative device in the form of a resilient plug;
FIGURE 11A illustrates an embodiment of the invention wherein a coiled wire is used to occlude a disc defect;
FIGURE 11B is a side-view representation of the coiled wire of Figure 11A;
FIGURE 11C illustrates how a wire with a coiled memory shape may be straightened and introduced using a plunger-type instrument;
FIGURE 12 although not within the scope of the invention illustrates a material in liquid or gel form may be introduced into a defect, after which it hardens or solidifies to prevent further rupturing;
FIGURE 13A although not within the scope of the invention illustrates a stent having a plurality of leaves;
FIGURE 13B although not within the scope of the invention illustrates the alternative of Figure 13A, wherein the leaves assume a second shape associated with defect occlusion, preferably through memory affect;
FIGURE 14A although not within the scope of the invention illustrates a conformable device is suspended within a gel or other resilient material for defect occlusion;
FIGURE 14B although not within the scope of the invention is a side-view drawing of Figure 14A;
FIGURES 15A-15E although not within the scope of the invention are drawings wherein a patch is used inside and/or outside of a void in need of occlusion;
FIGURE 16A although not within the scope of the invention is a top-view, cross-sectional drawing of utilizing posts or darts and sutures;
FIGURE 16B although not within the scope of the invention is a side-view drawing of Figure 16A;
FIGURE 17A although not within the scope of the invention shows how posts or darts may be criss-crossed to form a barrier;
FIGURE 17B although not within the scope of the invention is a side-view drawing of the configuration of Figure 17A;
FIGURE 18A although not within the scope of the invention is a side-view drawing of a barbed post that may be used for occlusion;
FIGURE 18B although not within the scope of the invention is an on-access view of the barbed post;
FIGURE 18C although not within the scope of the invention illustrates how a single larger barbed post may be used for defect occlusion;
FIGURE 18D although not within the scope of the invention illustrates how the barbed post of Figures 18A and 18B may be used in plural fashion to occlude a defect;
FIGURE 19A although not within the scope of the invention is a drawing which shows how shaped pieces may be inserted to close off an opening;
FIGURE 19B although not within the scope of the invention continues the progression of Figure 19A, with the pieces being pulled together;
FIGURE 19C although not within the scope of the invention illustrates the pieces of Figures 19A and 19B in a snapped-together configuration;
FIGURES 20A-20E although not within the scope of the invention are a progression of drawings which show how a shaped body may be held into place with one or more wires to block off a defect;
FIGURES 21A-21C although not within the scope of the invention illustrate how wires may be used in conjunction with snap-on beads to occlude a defect;
FIGURE 22A although not within the scope of the invention illustrates the insertion of members adapted to receive a dam component;
FIGURE 22B although not within the scope of the invention illustrates the dam of Figure 22A locked into position;
FIGURE 23A although not within the scope of the invention illustrates one form of defect · block that accommodates compression and distraction;
FIGURE 23B although not within the scope of the invention shows the device of Figure 23A in compression;
FIGURE 23C although not within the scope of the invention shows the device of Figure 23A in distraction;
FIGURE 23D although not within the scope of the invention illustrates the way in which the device, may be tacked into place with respect to upper and lower vertebrae;
FIGURE 24A although not within the scope of the invention is a drawing which shows an alternative device that adjusts for compression and distraction, in the form of a resilient dam,
FIGURE 24B although not within the scope of the invention shows the resilient dam in compression;
FIGURE 24C although not within the scope of the invention shows the resilient dam in distraction;
FIGURE 25 although not within the scope of the invention illustrates a different configuration for the insertion of a resilient dam according to the invention;
FIGURE 26 although not within the scope of the invention illustrates an alternative Z-shaped dam of resilient material;
FIGURE 27A although not within the scope of the invention illustrates the use of interlocking fingers that permit compression and distraction while occluding a defect;
FIGURE 27B although not within the scope of the invention is a side-view drawing in cross-section of the configuration of Figure 27;
FIGURE 28A although not within the scope of the invention illustrates an alternative interlocking finger configuration, and the way in which such members are preferably installed;
FIGURE 28B although not within the scope of the invention shows how the first of the multiple members of Figure 28A is installed;
FIGURE 29A although not within the scope of the invention is a side-view drawing ofa non-contained silicon blocking member prior to distraction;
FIGURE 29B although not within the scope of the invention illustrates the way in which the device of Figure 29A deforms upon distraction;
FIGURE 30A although not within the scope of the invention is a side-view drawing in cross-section illustrating a contained silicon structure prior to distraction; and
FIGURE 30B although not within the scope of the invention illustrates how the contained silicon structure of Figure 30A remains essentially the same in shape upon distraction.

### Detailed Description of the Invention

Having discussed the problems associated with post-operative partial discectomy with respect to Figures 1A-1C, reference will now be made to Figure 2, which illustrates a device in the form of a stent 202 is used to occlude a defect 204 in a human disc, as shown. The device is metallic. For reasons discussed below, a titanium mesh screen is preferred.

A flexible device is also preferred because the surgeon is presented with a very small working area. The incision through the skin is typically on the order of 1 to 1.5 inches in length, and the space at the disc level is approximately 1 centimeter on the side. As a consequence, the inventive device and the tools associated with insertion and fixation described below must be sufficiently narrow to fit within these confines.

As shown in Figures 3A-3C, a flexible screen enables the device to be collapsed into an elongated form 302, which, in turn, facilitates introduction into a sheath 304 associated with insertion. A push rod 306 may then be introduced into the other end of the sheath 304, and either the sheath pulled backwardly or the push rod pushed forwardly, or both, resulting in the shape shown in Figure 3C, now suitable for implantation.

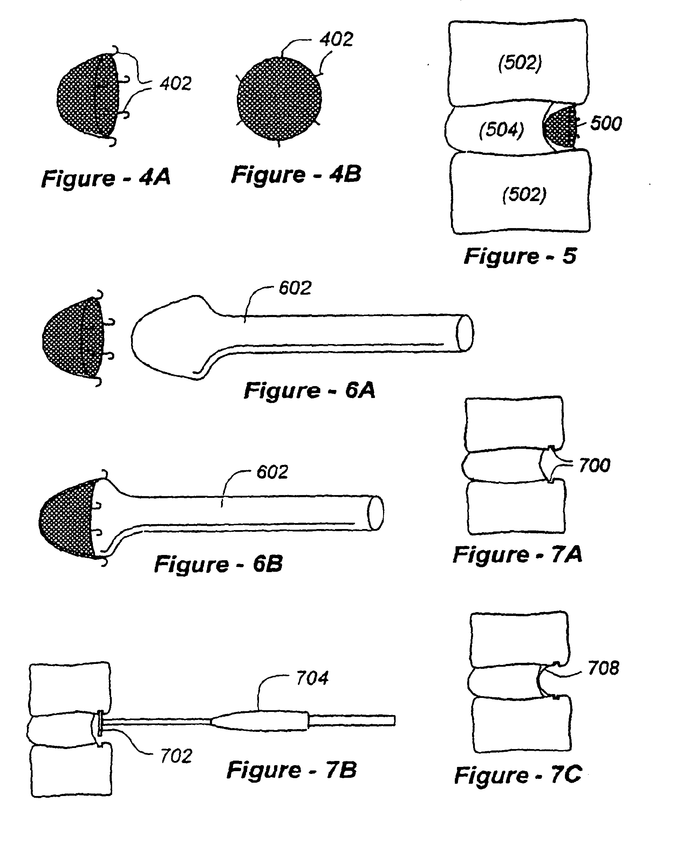

To further assist in fixation with respect to the surrounding physiology. anchors 402 may be provided around a peripheral edge of the device, as shown in Figure 4A. Figure 4B shows an end view of the device of Figure 4A, and Figure 5 illustrates the device with anchors generally at 500, being fixed relative to a defective disc 504 bounded by upper and lower vertebrae at 502. It will be apparent to those of skill that each of the devices disclosed herein may be made in different sizes, having varying peripheral dimensions, for example, to match differently sized defects.

Figure 6A and 6B illustrate how a distracting tool 602 may be used to force the anchors into the vertebrae. That is, having introduced the device into the approximate area, the tool 602, having a forward shape corresponding to that of the expanded mesh shape, may be introduced therein, as shown in Figure 6B. With force being applied to the tool 602. the anchors may be permanently set into the surrounding bone/ tissue.

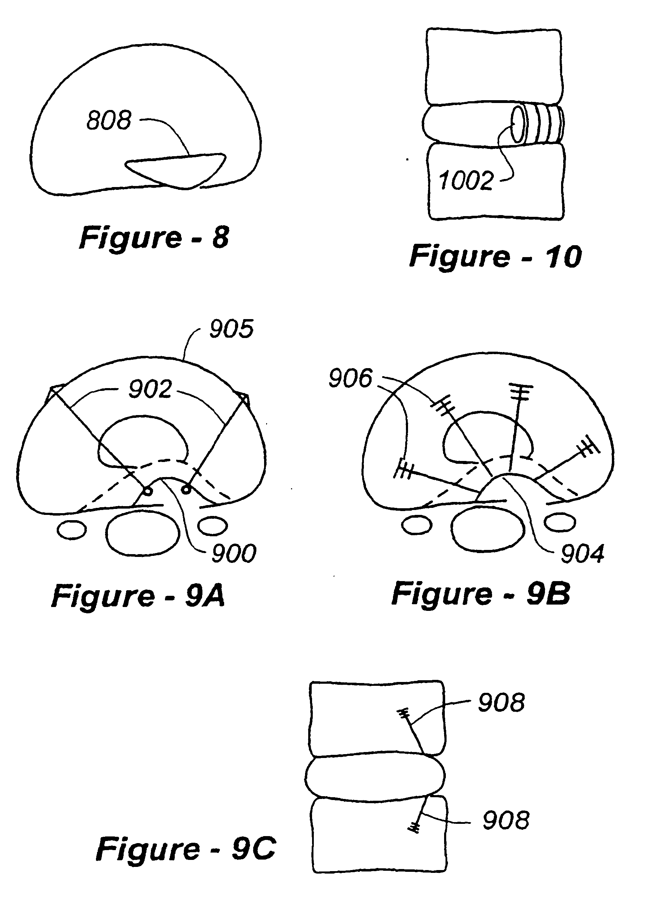

Figure 7A illustrates an alternative approach to fixation, wherein one or more notches 700 may be made into the upper and lower vertebra, preferably through the use of an air-operated drill 704 shown in Figure 7B, having a cutting wheel 702 adapted for such a purpose. Figure 7C illustrates the way in which a flexible body 708 may be retained by the notches 700 described with respect to Figures 7A and 7B. Figure 8 illustrates an alternative orientation of a flexible body having a convex surface facing outwardly with respect to the wall of the disc being repaired.

Figure 9A illustrates a further alternative associated with fixation wherein anchors 902 which penetrate the outer wall of the disc 905 are used to hold a flexible repair device 900 in place as shown. Figure 9B shows yet a further alternative fixation modality, wherein disc anchors 906, which do not penetrate the outer wall of the disc, but, rather remain there within, are used to hold the device 904 in place.

Figure 9C illustrates yet a further alternative mode of fixation, wherein anchors 908 are used to hold the device to upper and lower vertebra, as opposed to the anchors of Figures 9A and 9B, which are used with respect to the disc. Regardless of whether fixation takes place within the vertebra or within the disc. it will be noted that according to the preferred embodiment of the invention, both the device used to occlude the defect and the fixation means are sufficiently flexible that the defect remains occluded with movement ofthe spine, that is, with the patient leaning forwardly and backwardly which will tend to change the spacing between the upper and lower vertebra.

Figure 10 although not within the scope of the invention illustrates, as opposed to a piece of flexible material or mesh, a resilient plug 1002 is instead utilized to occlude the disc defect. As in the case of the flexible sheath-like embodiments described above, such plugs are preferably offered in different sizes to correlate with differently sized defects.

In terms of a preferred material, a device will therefore remain sufficiently flexible during movement while being capable of exerting continuous outward forces and withstanding repetitive compression and distraction of millions of cycles. The device would, therefore, preferably be made of a material that has these characteristics, while, additionally being radio-opaque for X-ray imaging, without producing too many unwanted artifacts in magnetic resonance imaging. A wire mesh of titanium is therefore preferable, since this has the proper X-ray/MRI characteristics while exhibiting the requisite flexibility for the cyclic flexion and extension.

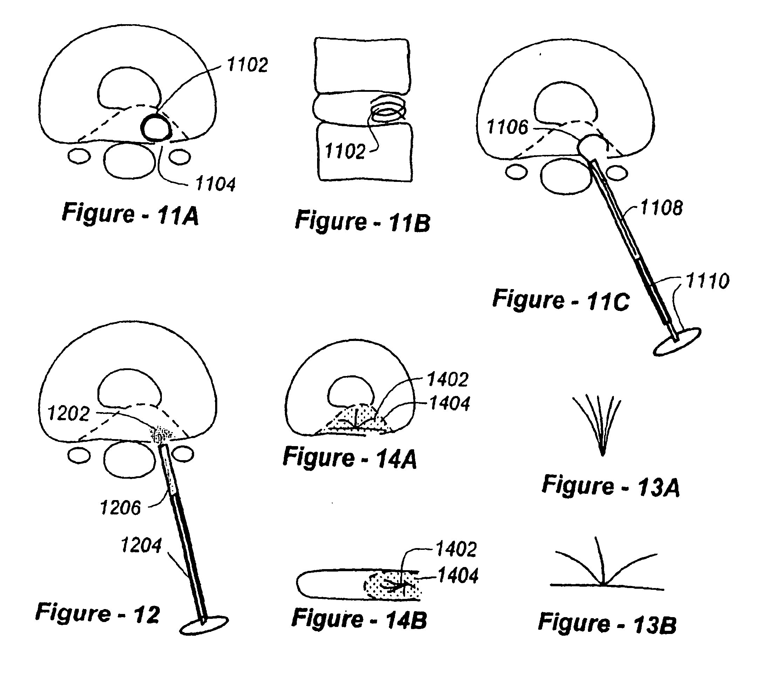

The invention is as defined in claim 1. As shown in Figures 11A-11C, for example, a wire 1102 having a "memory effect" may be used, preferably having a final diameter which is larger than void 1104. Figure 11B shows the coil 1102 in cross-section between upper and lower vertebra. Preferably, this embodiment would use a metal wire that may be straightened, but retain the memory of its coiled shape. As such, the apparatus of Figure 11C may be used to introduce the wire in straightened form 1108 with a plunger 1110, such that as the wire exits at 1106, it returns to its memorized state of a coil (or alternative second shape operative to include the defect).

As yet a different alternative mode of introduction, a material may be injected into the disc in liquid form, then allowed to hardened into a size sufficient to occlude the annular hole. As shown in Figure 12 although not within the scope of the invention, material 1202 may be injected into the void of the disc space using a plunger 1204 inserted into a tube 1206. Upon introduction in this manner, the liquid would then solidify, forming a resilient plug.

Various materials may be utilized for this purpose, including various polymers which are caused to solidify by various means. including thermal or optical activation, or chemical reaction as part of multi-part compounds. A preferred material with respect to this would be a hydrogel. Hydrogels may be placed into the disc in a dehydrated state, and, once inside the disc, they imbibe water. After hydration, hydrogels have the same biomechanical properties as a natural nucleus and, in addition, as the hydrogels swell, they become too large to extrude back through the annular window. Patent Nos. 5,047,055 and 5,192,326 provide a listing of hydrogels, certain ofwhich are applicable.

An elastomer may be used as an alternative to a hydrogel or other material. A n umber of elastomers may be suited, including a silicon elastomer, which comprises a cured dimethylsiloxane polymer and Hexsyn, having a composition of one-hexane with three to five percent methylhexaiene. A preformed elastomer may be inserted into the inclusion upon curing or, alternatively, as discussed with reference to Figure 12, may be injected into the disc space and liquid form. Chemicals may be added to accelerate curing, as discussed above, or, a hot or cold probe, or UV light may be introduced to facilitate or accelerate the curing process. Preferably, such materials would include a radio-opaque additive which would enable the physician to verify the position of the implant with an X-ray. Ideally, the radio-opaque additive would not change the mechanical properties of the gel or elastomer, and would ideally incorporate contrast throughout to enhance detail.

Now making to Figures 13 and 14, Figures 13A and 13B illustrate an alternative type of stent having leaves or other appendages that may be folded into a compact state for insertion, Figure 13A, and which expand, through memory affect, for example, to a state such as that shown in Figure 13B. A stent such as this, as well as other devices disclosed herein such as the coil form of Figure 11, may be used in conjunction with a gel or other void-filling material as described above. As shown in Figure 14A, a stent 1402 of the type shown with respect to Figure 13B, may be introduced into the void, after which the remaining volume of the void may be filled with a material 1404 which solidifies into a resilient material. Figure 14B is a side-view drawing of Figure 14A. An expandable stent of this kind may be incorporated into the elastomer or other resilient material to help prevent migration of the prosthesis through the annular hole. In contrast wherein a stent is used independently, the stent would preferably not touch vertebra, since it would be surrounded entirely by the elastomer or other gel material.

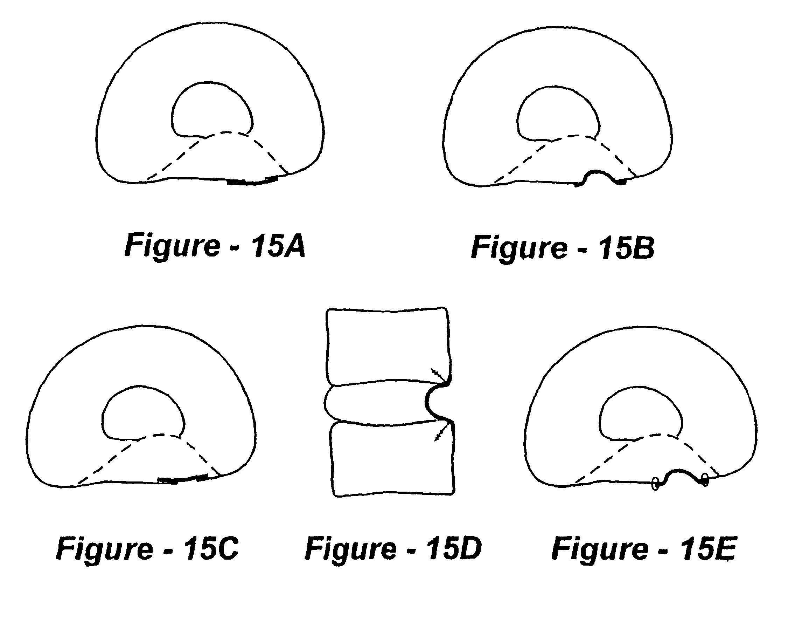

Figures 15A-15E illustrate patch material used inside, outside, or partially inside and outside of a defect to be blocked. Figure 15A illustrates a flat patch attached onto the outside of the disc. Figure 15B illustrates a patch attached on the outside but wherein a central portion extends inwardly into the void. Figure 5C illustrates a patch disposed within the disc to block the defect. Figure 15D illustrates how a patch may be anchored to the bone above and below the disc, and Figure 15E illustrates how the patch may be anchored to the disc itself. The patch may be attached within appropriate means, including stitches, staples, glue, screws or other special anchors.

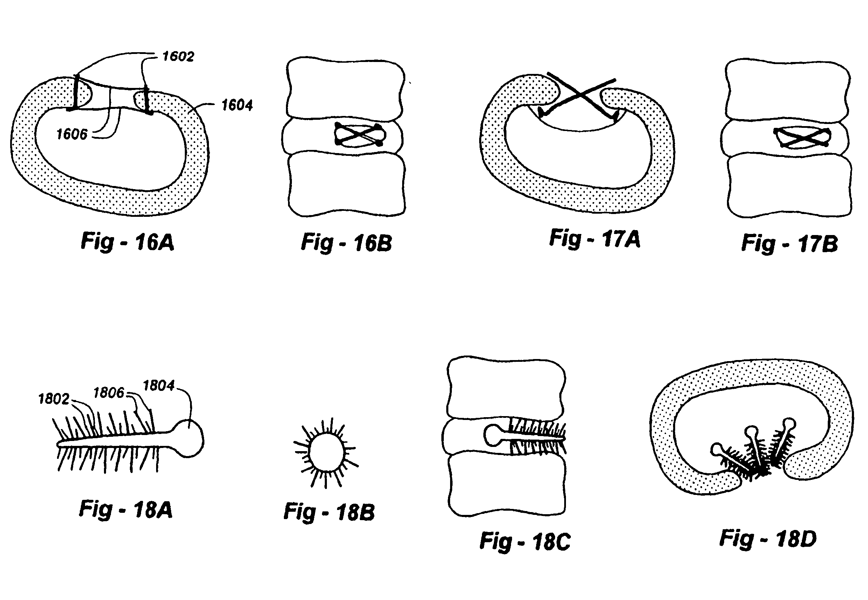

In addition to the use of patches attached with sutures, staples or other materials, the annular defect may be closed with staples or other devices which attach to the annulus without the need for patch material. For example, as shown in Figure 16A, darts 1602 may be inserted through the wall of the annulus 1604, then linked with sutures 1606, preferably in woven or criss-crossed fashion, as shown in Figure 16B. As an alternative, appropriately shaped darts 1702 may be criss-crossed or otherwise interlocked to the close the annular hole, as shown in the top-view cross-section drawing of Figure 17A or a side-view of Figure 17B.

The use of flexible stents as described elsewhere herein may take on other forms, as shown in Figures 18A-18D. The device of Figure 18A, for example, preferably includes a body 1802, preferably including a blunt anterior end to prevent penetration of the anterior annulus, and outer spikes 1806, preferably having different lengths, as best seen in the on-axis view of Figure 18B. Such a stent configuration may provide more areas of contact with the vertebral end plates, thereby decreasing the chances of stent extrusion. As shown in Figure 18C, the longer spikes 1806 are configured to bend during insertion, thereby preventing posterior extrusion. The shorter spikes, 1806', are sized so as not to engage the vertebrae, and therefore may be made thicker to prevent deflection by disc material. As an option, the shorter spikes 1806' may also be angled in the opposite direction as compared to the longer spikes 1806 to resist migration of the disc material. As yet a further option, the longer spikes may vary in length on the same stent so as to be conformal to the vertebral end plate concavity. As shown in Figure 18D, multiple spike stents of this kind may be inserted so as to interlock with one another, thereby preventing migration of the group.

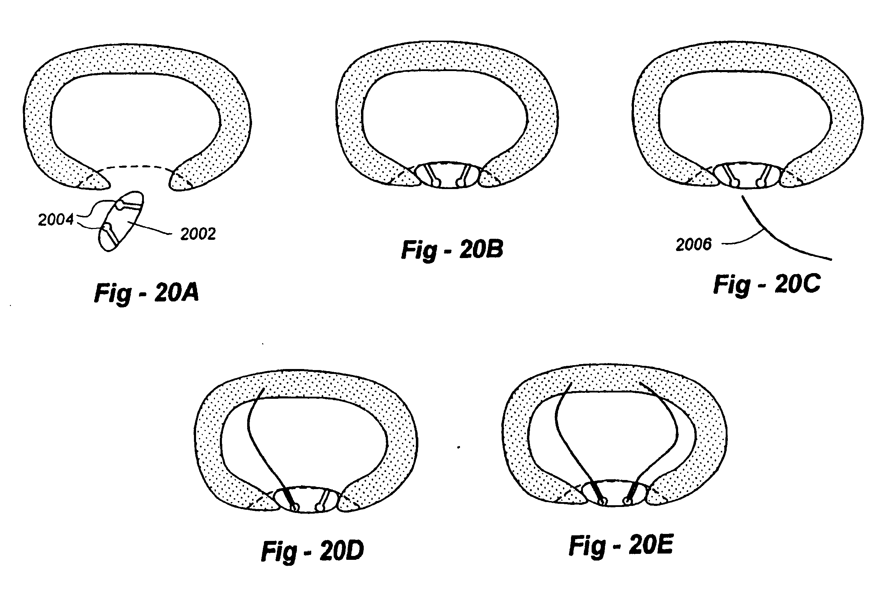

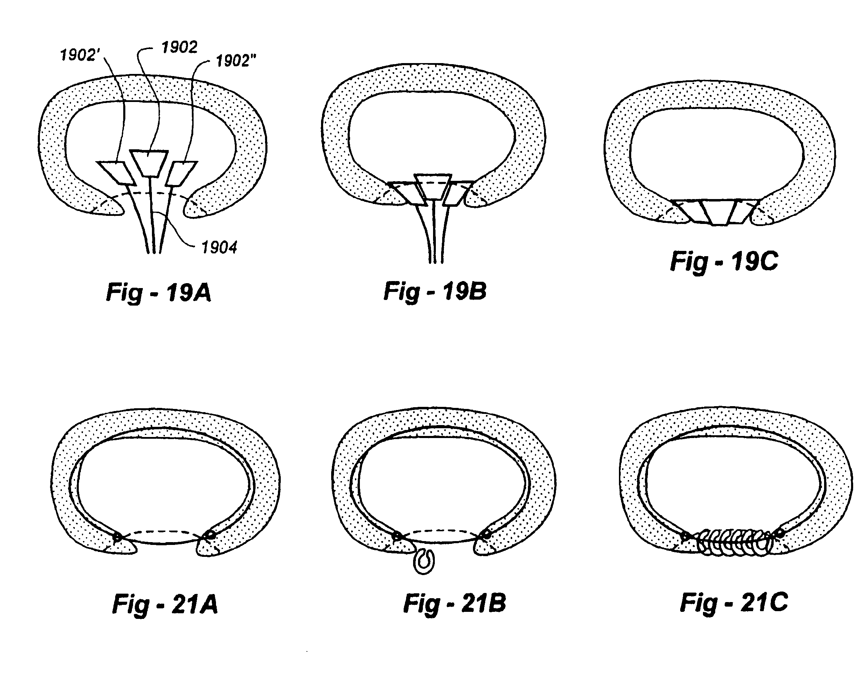

As shown in Figures 19A-19C although not within the scope of the invention, shapes other than spiked stents may be used in interlocking fashion. In Figure 19A, a first piece 1902 is inserted having a removable handle 1904, after which pieces 1902' and 1902" are inserted, each having their own removable handles, as shown. In Figure 19B, the handles are pulled. so as to bring the pieces together, and in Figure 19C, the handles are removed, and the pieces are either snapped together or, through the use of suitable material, sutured into place. Figures 20A-20E illustrate a different configuration of this kind, wherein a body 2002 having anchor or wire-receiving apertures 2004 is inserted into the annular hole, as shown in Figure 20B. at which time a wire 2006 is inserted through the body 2002 as shown in Figure 20C. As shown in Figure 20D, the wire is installed sufficient to lock one portion of the body into place. and this is followed with a wire on the opposite side, thereby holding the body 2002 in a stabilized manner. It will be appreciated that although multiple wires or anchors are used in this configuration, bodies configured to receive more or fewer wires or anchors are also anticipated by this basic idea.

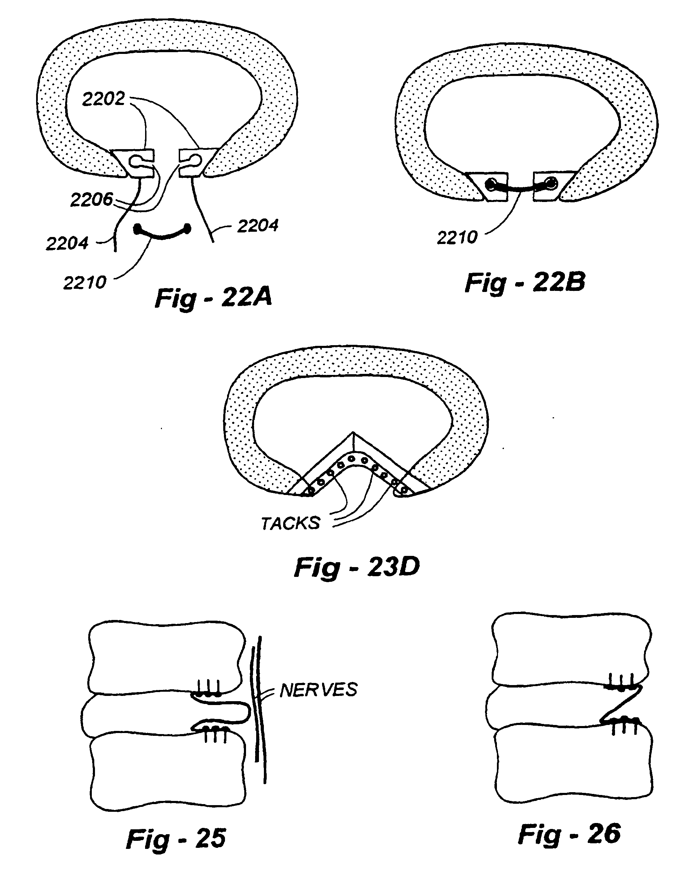

Figures 21A-21C illustrate a different alternative, wherein wires 2102 each having a stop 2104 are first inserted through the annular window. after which blocking beads having snap-in side configurations are journaled onto the wire across the annular hole, as shown in Figure 21B. Figure 21C illustrates how, having locked multiple beads onto the wire, the defect is affectively occluded. Figures 22A and 22B illustrate the use of a removable dam component. As shown in Figure 22A, bodies 2202, each having removable handles 2204, are first inserted on the side portions of the defect, each member 2202 including slots, grooves or apertures 2206, configured to receive a dam 2210, which may be made of a rigid or pliable material, depending upon vertebral position, the size of the defect, and other factors. Figure 22B illustrates the dam 2210 locked in position.

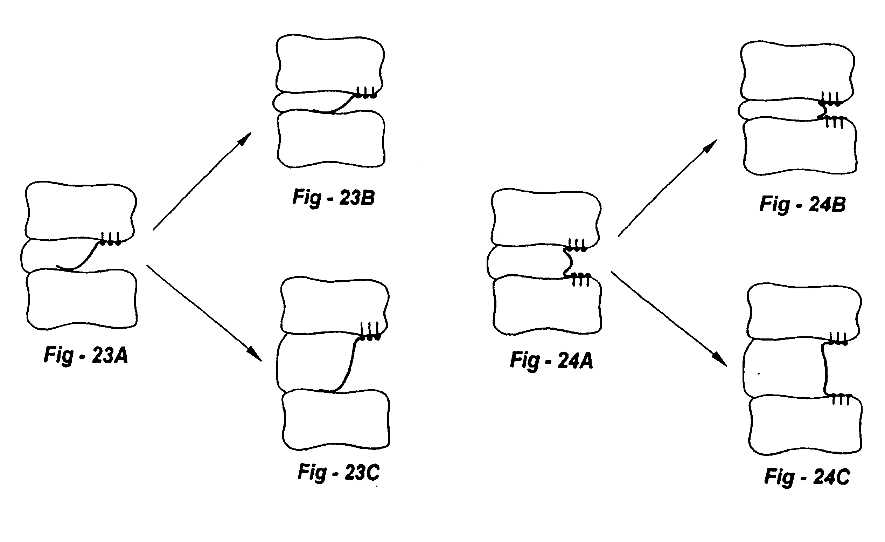

In Figure 23A, for example. a flexible element 2302 is tacked into position on the upper vertebrae, as perhaps best seen in Figure 23D, though it should be apparent that a fixation to the lower vertebrae may also be used. Figure 23B illustrates how, once the member 2302 is fastened in place, it may flex under compression, but return to a more elongated shape in distraction, as shown in Figure 23C. The blocking element 2302 is made from various shape-memory materials, so long as it performs the function as described herein. Figure 24A illustrates a different configuration, which is tacked to both the upper and lower vertebrae, and Figures 24B and 24C show how the device performs in compression and distraction, respectively.

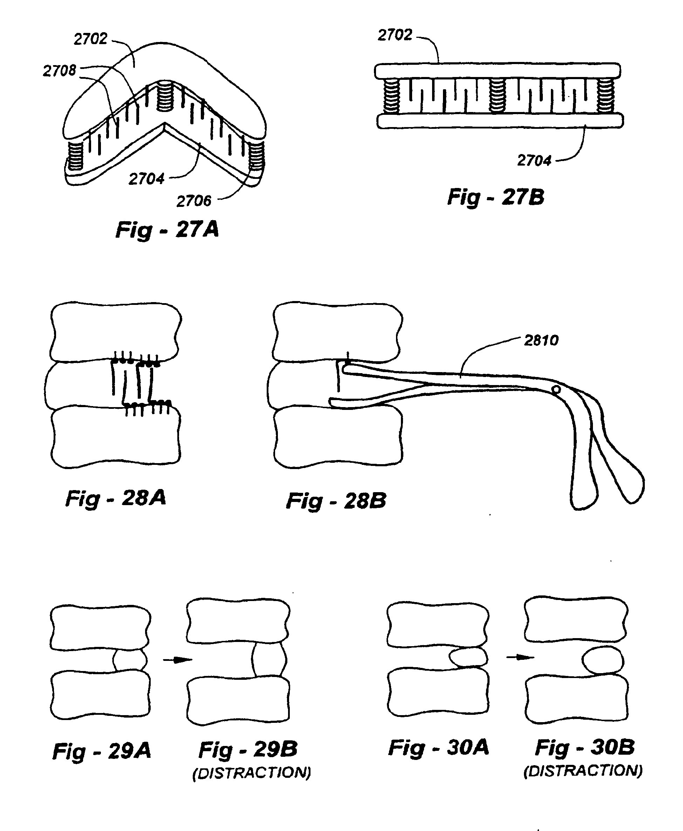

As an alternative to inherently flexible materials which occlude a defect while accommodating compression and distraction, interleaving members may alternatively be used, as shown in Figures 27-28 although not within the scope of the invention. Figure 27A is a view from an oblique perspective, showing how upper and lower plate 2702 and 2704 of any suitable shape, may be held together with springs 2706, or other resilient material, between which there is supported interleaving tines 2708. As better seen in Figure 27B, the springs 2706 allow the upper and lower plates 2702 and 2704 to move toward and away from one another, but at all times, tines 2708 remain interleaving, thereby serving to block a defect.

Figures 28A and 28B illustrate the way in which interleaving members or tines are preferably inserted directly to vertebrae. Since each member overlaps with the next, such tines are preferably installed from front to back (or back to front. as the case may be), utilizing a tool such as 2810, as shown in Figure 28B. The instrument 2810 forces each tack into one vertebrae at a time by distracting against the other vertebrae, thereby applying pressure as the jaws are forced apart, driving the tack into the appropriate vertebrae. The tack may be held into place on the instrument by a friction fit. and may include a barbed end so as not to pull out following insertion.

As a further alternative configuration although not within the scope of the invention, a collapsed bag may be placed into the disc space, then filled with a gas, liquid or gel once in position. The bag may be empty, or may contain a stent or expanding shape to assist with formation. In the case of a gel, silicon may be introduced so as to polymerized or solidify. As shown in Figures 29A and 29B, the use of a non-contained silicon vessel may be used, but, under distraction, may remain in contact with the vertebrae, thereby increasing the likelihood of a reaction to silicone. Figure 30A utilizes a contain structure in the case of a silicon filler, as shown in Figure 30A, such that, upon distraction. the vessel remains essentially the same shape, thereby minimizing vertebral contact.

## Claims

1. A device for preventing the escape of natural, artificial or therapeutic material through a defective region in an annulus fibrosis of a spinal disc, and for preventing disc herniation,
said device having a straightened first physical extent facilitating introduction of the device relative to the defective region in the annulus fibrosis, and
a memorised second physical extent forming a final shape of the device, different from the first, **characterised in that** the device in the memorised second physical extent is a coiled metal wire composed of memory effect metal material that naturally returns to the memorised second physical extent, and which functions to occlude the defective region by expanding from the first physical extent to the second physical extent,
wherein no further steps are required to form the memorised second physical extent.

## Patentansprüche

1. Vorrichtung zum Verhindern des Austretens von natürlichem, künstlichem oder therapeutischem Material durch einen defekten Bereich in einem *Annulus fibrosis* einer Bandscheibe und zum Verhindern eines Bandscheibenvorfalls,
wobei die Vorrichtung eine geradegerichtete erste körperliche Erstreckung aufweist, die das Einführen der Vorrichtung in Bezug auf den defekten Bereich in dem *Annulus fibrosis* erleichtert, und
eine memorisierte zweite körperliche Erstreckung, die eine endgültige Form der Vorrichtung bildet, welche sich von der ersten unterscheidet, **dadurch gekennzeichnet, dass** die Vorrichtung in der memorisierten zweiten körperlichen Erstreckung ein gewickelter Metalldraht ist, der aus Memory-Effekt-Metallmaterial zusammengesetzt ist, das von Natur aus zu der memorisierten zweiten körperlichen Erstreckung zurückkehrt, und die dazu dient, den defekten Bereich durch Ausdehnen von der ersten körperlichen Erstreckung zu der zweiten körperlichen Erstreckung zu verschließen,
wobei keine weiteren Schritte erforderlich sind, um die memorisierte zweite körperliche Erstreckung zu bilden.

## Revendications

1. Dispositif pour empêcher la fuite d'un matériau naturel, artificiel ou thérapeutique à travers une région défectueuse dans une fibrose annulaire d'un disque vertébral, et pour empêcher l'apparition d'une hernie au niveau du disque,
ledit dispositif ayant une première étendue physique redressée facilitant l'introduction du dispositif par rapport à la région défectueuse dans la fibrose annulaire, et
une deuxième étendue physique mémorisée formant une forme définitive du dispositif, différente de la première, **caractérisé en ce que** le dispositif dans la deuxième étendue physique mémorisée est un fil de métal hélicoïdal composé d'un matériau métallique à mémoire de forme qui revient naturellement à la deuxième étendue physique mémorisée, et qui fonctionne pour boucher la région défectueuse en s'expansant de la première étendue physique à la deuxième étendue physique,
dans lequel aucune étape supplémentaire n'est nécessaire pour former la deuxième étendue physique mémorisée.
